(19) Europäisches Patentamt
European Patent Office
Office européen des brevets

(11) Publication number: **0 313 317 B1**

(12) **EUROPEAN PATENT SPECIFICATION**

(45) Date of publication of patent specification: **22.04.92**

(51) Int. Cl.[5]: **A01N 47/36**, A01N 25/04

(21) Application number: **88309772.7**

(22) Date of filing: **19.10.88**

(54) **Herbicidal suspension concentrate.**

(30) Priority: **22.10.87 JP 267548/87**

(43) Date of publication of application:
**26.04.89 Bulletin 89/17**

(45) Publication of the grant of the patent:
**22.04.92 Bulletin 92/17**

(84) Designated Contracting States:
**DE ES FR GB GR IT**

(56) References cited:
**JP-A- 6 130 585**

(73) Proprietor: **ISHIHARA SANGYO KAISHA, LTD.
No. 3-22, Edobori 1-chome
Nishi-ku Osaka(JP)**

(72) Inventor: **Ogawa, Yasuo Ishihara Sangyo
K.K. Chuo Kenkyusho 3-1, Nishishibukawa
2-chome
Kusatsu-shi Shiga-ken(JP)**
Inventor: **Kimura, Fumio Ishihara Sangyo
K.K. Chuo Kenkyusho 3-1, Nishishibukawa
2-chome
Kusatsu-shi Shiga-ken(JP)**
Inventor: **Kimura, Akira Ishihara Sangyo
K.K. Chuo Kenkyusho 3-1, Nishishibukawa
2-chome
Kusatsu-shi Shiga-ken(JP)**
Inventor: **Hiratsuka, Takeshi Ishihara Sangyo
K.K. Chuo Kenkyusho 3-1, Nishishibukawa
2-chome
Kusatsu-shi Shiga-ken(JP)**
Inventor: **Sakashita, Nobuyuki Ishihara Sangyo
K.K. Chuo Kenkyusho 3-1, Nishishibukawa
2-chome
Kusatsu-shi Shiga-ken(JP)**
Inventor: **Honda, Chimoto Ishihara Sangyo
K.K. Chuo Kenkyusho 3-1, Nishishibukawa
2-chome
Kusatsu-shi Shiga-ken(JP)**

(74) Representative: **Pearce, Anthony Richmond et al
MARKS & CLERK Alpha Tower Suffolk Street
Oueensway Birmingham B1 1TT(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a herbicidal suspension concentrate comprising a specific sulfonamide compound(s) and/or a salt(s) thereof, a vegetable oil, and a surfactant at a specified ratio. This is a useful concentrate which can control a wide variety of harmful weeds without any phytotoxicity against corns when applied to corn fields.

European Patent-A-232,067 and 237,292 both disclose that sulfonamide compounds and their salts, which are the active ingredients contained in the herbicidal suspension concentrate of the present invention, are useful as herbicides for application to corn fields. However, they fail to disclose that these compounds can be admixed with a vegetable oil and a surfactant, thereby to prepare a suspension concentrate.

While, on the other hand, U.S. Patent No. 3,997,322, U.S. Patent No. 4,529,438, British Patent No. 803,772, West German Patent No. 2,701,129, Chemical and Engineering News, July 21, 1969, page 41, Japanese Patent Application No. 58-8232 (published on August 2, 1984 as publication No. KOKAI 59-134702) and Japanese Patent Application No. 58-242982 (published on July 16, 1985 as publication No. KOKAI 60-132904) all contain descriptions relating to the preparation of herbicidal compositions by mixing herbicidal compounds with vegetable oils, these prior art papers, however, fail to disclose that the specific sulfonamide compounds and their salts which are contained in the herbicidal suspension concentrate of the present invention can be used as herbicidal compounds.

The present invention relates to a herbicidal suspension concentrate characterized by comprising from 0.5 to 20 parts by weight of at least one compound selected from among sulfonamide compounds represented by the general formula (I):

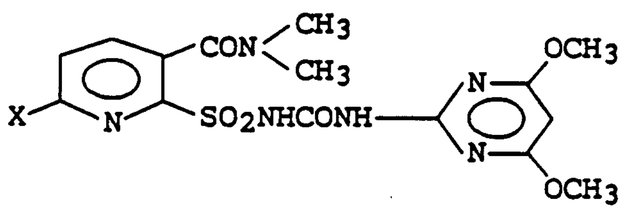

(I)

(wherein X is a hydrogen atom, a chlorine atom, a bromine atom, a methyl group, or a difluoromethyl group) and their salts as an active ingredient, from 55 to 94.5 parts by weight of a vegetable oil, and from 5 to 25 parts by weight of a surfactant.

The suspension concentrate of the present invention, when applied to a corn field, provides improved weeding effect against harmful weeds in general, and against gramineous weeds in particular, without any phytotoxicity against corns. As a result of this improvement, the spectra of the harmful weeds to be controlled are spread, and further the amount of the active ingredient compound to be used can be decreased.

As was mentioned previously, the sulfonamide compounds of the present invention, represented by the general formula (I), and their salts are already known as herbicides for application to corn fields. In practical use of these compounds, however, it is still required to decrease the amount of the active ingredient compound to be used in view of the reduction of expenditure for weeding and the reduction of environmental contamination. While it is also required to control as perfectly as possible a variety of weeds differing in growth stage without any crop injury to corns.

The authors of the present invention have found that use of a vegetable oil in the preparation of a suspension concentrate wherein the sulfonamide compound, represented by the general formula (I), or its salt is blended with the vegetable oil and a specific surfactant at a specific weight ratio can satisfy the requirements mentioned above when an aqueous diluted suspension prepared from the suspension concentrate is applied to a corn field. Namely they have found that use of the vegetable oil improves the weeding effect against broad spectra of the harmful weeds to be controlled without any phytotoxicity against corns and can decrease the amount of the active ingredient compound to be used, and that consequently reduction of environmental pollution can be expected.

Examples of sulfonamide compounds which are represented by the general formula (I), and which therefore can be used in the present invention, include N-[(4,6-dimethoxypyrimidin-2-yl)aminocarbonyl]-3-dimethylaminocarbonyl-2-pyridinesulfonamide, N-[(4,6-dimethoxypyrimidin-2-yl)aminocarbonyl]-6-chloro-3-dimethylaminocarbonyl-2-pyridinesulfonamide, N-[(4,6-dimethoxypyrimidin-2-yl)aminocarbonyl]-6-bromo-3-dimethylaminocarbonyl-2-pyridinesulfonamide, N-[(4,6-dimethoxypyrimidin-2-yl)aminocarbonyl]-3-dimethylaminocarbonyl-6-methyl-2-pyridinesulfonamide, and N-[(4,6-dimethoxypyrimidin-2-yl)-

aminocarbonyl]-6-difluoromethyl-3-dimethylaminocarbonyl-2-pyridine-sulfonamide.

Further, salts of the above-mentioned sulfonamide compound include those of alkali metals such as sodium and potassium, those of alkali earth metals such as magnesium and calcium, and those of amines such as monomethylamine, dimethylamine and triethylamine, specific examples of which include sodium and monomethylamine salts of N-[(4,6-dimethoxypyrimidin-2-yl)aminocarbonyl]-3-dimethylaminocarbonyl-2-pyridine-sulfonamide, and dimethylamine salt of N-[(4,6-dimethoxypyrimidin-2-yl) aminocarbonyl]-3-dimethyl-aminocarbonyl-6-methyl-2-pyridinesulfonamide.

Among the above-mentioned sulfonamide compounds, N-[(4,6-dimethoxypyrimidin-2-yl)aminocarbonyl]-3-dimethylaminocarbonyl-2-pyridinesulfonamide and its salts are exemplified as preferred compounds.

Examples of vegetable oils which can be used in the present invention include olive oil, kapok oil, castor oil, palm oil, camellia oil, coconut oil, sesame oil, corn oil, rice bran oil, peanut oil, cotton seed oil, soybean oil, rape seed oil, linseed oil, and tung oil. Among these vegetable oils, corn oil is particularly preferred.

In the present invention, any surfactant may be used as long as it can emulsify the above-mentioned vegetable oils in water.

Examples of such surfactants include those of vegetable oil derivatives such as polyoxyethylene hydrogenated castor oil ether; nonionic surfactants such as polyoxyethylene alkyl ethers, polyoxyethylene alkylaryl ethers, polyoxyethylene (propylene)fatty acid esters, sorbitan monooleate, and polyoxyethylene sorbitan monolaurate; and anionic surfactants such as sodium alkylarylsulfonates, sodium dialkylsuccinate sodium dialkylsulfosuccinates, polyoxyethylene alkylaryl ether sulfates, polyoxyethylene alkyl phosphates, polyoxyethylene alkylaryl phosphates, and sodium alkylnaphthalenesulfonates.

Particularly, a mixture of a surfactant of vegetable oil derivatives, a nonionic surfactant and an anionic surfactant is preferably used.

The suspension concentrate of the present invention is prepared, for example, according to the following procedure.

At least one compound selected from among the sulfonamide compounds represented by the aforementioned general formula (I) and their salts as an active ingredient compound(s) is uniformly mixed with a vegetable oil and a surfactant and the resulting mixture is ground in order to obtain a suspension concentrate. Alternatively, such an active ingredient compound(s) preliminarily ground is merely mixed with a vegetable oil and a surfactant to obtain a suspension concentrate.

When consideration is given to the suspension stability of the active ingredient compound(s) in the suspension concentrate, a thixotropic material(s) such as a bentonite-alkylamine complex(es) and/or aerosil may be added in an amount of, for example, 1 to 3 weight % based on the total concentrate if necessary.

The suitable blending weight ratio of the above-mentioned active ingredient compound(s), the vegetable oil and the surfactant, which are the main components composing the suspension concentrate of the present invention, is in the ranges of from 0.5 to 20:from 55 to 94.5:from 5 to 25 respectively, and preferably from 2 to 6:from 77 to 90:from 8 to 17 respectively.

The suitable amount of the suspension concentrate of the present invention to be used, cannot be generically specified because it varies depending on various conditions. In general, however, it is in the range of from 0.05 to 50 g/a, preferably from 0.1 to 25 g/a, and more preferably from 0.1 to 2.5 g/a, in terms of the amount of the active ingredient compound.

In order to illustrate the present invention in more detail, description will now be made of Formulation Examples wherein the herbicidal suspension concentrates of the present invention were prepared and Test Examples wherein some of the prepared suspension concentrates were used, both of which, however, should not be construed as limiting the scope of the present invention.

## Preparation of Suspension Concentrates

### Formulation Example 1

(1) N-[(4,6-dimethoxypyrimidin-2-yl)aminocarbonyl]-3-dimethylaminocarbonyl-2-pyridinesulfonamide 5 parts by weight

(2) corn oil 84 parts by weight

(3) Sorpol 3747 (trade name of a mixture of a polyoxyethylene alkylaryl ether, polyoxyethylene hydrogenated castor oil ether, a polyoxyethylene alkylaryl phosphate, a sodium dialkyl-sulfosuccinate, and a fatty acid manufactured by Toho Chemical Co., Ltd.) 10 parts by weight

(4) bentonite-alkylamine complex 1 part by weight

The above-mentioned components (1) to (4) were mixed uniformly and the resulting mixture was ground with a Dyno-Mill (manufactured by Willy A. Bachofen AG) to obtain a suspension concentrate according to the present invention.

Formulation Example 2

A suspension concentrate according to the present invention was prepared in substantially the same manner as in the above-mentioned Formulation Example 1 except that 82 parts by weight of corn oil was used instead of 84 parts by weight and 12 parts by weight of Sorpol 3815 (trade name of a mixture of a polyoxyethylene alkylaryl ether, polyoxyethylene hydrogenated castor oil ether, a fatty acid derivative, and a sodium dialkylsulfosuccinate manufactured by Toho Chemical Co., Ltd.) was used instead of 10 parts by weight of Sorpol 3747.

Formulation Example 3

| | |
|---|---|
| (1) N-[(4,6-dimethoxypyrimidin-2-yl)aminocarbonyl]-3-dimethylaminocarbonyl-2-pyridinesulfonamide | .06 parts by weight |
| (2) corn oil, soybean oil, cotton seed oil, rape seed oil or linseed oil | 80.94 parts by weight |
| (3) Sorpol 3815 | 12 parts by weight |
| (4) bentonite-alkylamine complex | 2 parts by weight |

A suspension concentrate according to the present invention was prepared using the above-mentioned components (1) to (4) in substantially the same manner as in the above-mentioned Formulation Example 1.

Formulation Example 4

| | |
|---|---|
| (1) N-[(4,6-dimethoxypyrimidin-2-yl)aminocarbonyl]-3-dimethylaminocarbonyl-2-pyridinesulfonamide | 2 parts by weight |
| (2) corn oil | 85 parts by weight |
| (3) Sorpol 3815 | 10 parts by weight |
| (4) bentonite-alkylamine complex | 3 parts by weight |

A suspension concentrate according to the present invention is prepared using the above-mentioned components (1) to (4) in substantially the same manner as in the above-mentioned Formulation Example 1.

Formulation Example 5

| | |
|---|---|
| (1) N-[(4,6-dimethoxypyrimidin-2-yl)aminocarbonyl]-3-dimethylaminocarbonyl-2-pyridinesulfonamide | 6 parts by weight |
| (2) corn oil | 80 parts by weight |
| (3) Sorpol 3747 | 13 parts by weight |
| (4) bentonite-alkylamine complex | 1 part by weight |

A suspension concentrate according to the present invention is prepared using the above-mentioned components (1) to (4) in substantially the same manner as in the above-mentioned Formulation Example 1.

Plant Test Method and Results

Test Example 1

1/3,000 are (a) pots and 1/10,000 are (a) pots were filled with upland soil. Corn (Zea mays) (variety: Royal Dent 105 T) was sown in the 1/3,000 are (a) pots, while large crabgrass (Digitaria adscendens) and smartweed (Polygonum hydropiper) were separately sown in the 1/10,000 are (a) pots. When the plants reached respective given growth stages (a 4-leaf stage for corn, a 3-leaf stage for large crabgrass, and a 1-leaf stage for smartweed), 5 liters/a each of aqueous diluted suspensions, prepared by suspending in water the suspension concentrate prepared in the aforementioned Formulation Example 2 so as to provide respective amounts of the active ingredient of 1.25 g/a and 0.625 g/a, were each separately foliarly applied to the plants with a small spray gun.

The progress of growth of the plants was visually observed and examined 29 days after the application to evaluate the degree of growth control according to 10 ratings (1: the same as in an untreated plot to 10: perfect growth control). The results are shown in Table 1.

For comparison, a test was made using substantially the same procedure as in the above-mentioned Test Example 1 except that use was made of individual comparative suspensions prepared by suspending the following wettable powder in water so as to provide respective amounts of its active ingredient of 1.25 g/a and 0.625 g/a and admixing 0.2 weight % of Shin Rino (trade name of a mixture of a polyethylene glycolalkylphenol ether and a lignosulfonate manufactured by Nihon Nohyaku Co., Ltd.) with the respective resulting aqueous diluted suspensions. The results are shown in Table 1.

Table 1

| | Degree of Growth Control | | | | | |
|---|---|---|---|---|---|---|
| | corn 4L | | large crabgrass 3L | | smartweed 1L | |
| | Amount of Active Ingredient (g/a) | | | | | |
| | 1.25 | 0.625 | 1.25 | 0.625 | 1.25 | 0.625 |
| Plots Using Present Invention | 1 | 1 | 10 | 9 | 10 | 10 |
| Plots Using Comparative Suspension | 1 | 1 | 9 | 6-7 | 10 | 10 |

(Note) The wettable powder used in the comparative plots is a mixture composed of 40 parts by weight of N-[(4,6-dimethoxypyrimidin-2-yl)aminocarbonyl]-3-dimethylaminocarbonyl-2-pyridinesulfonamide, 8 parts by weight of Carplex #80 (trade name of synthetic fine silica manufactured by Shionogi & Co., Ltd.), 46 parts by weight of jeaklite, 4 parts by weight of Sorpol 5039 (trade name of a polyoxyethylene alkylaryl ether sulfate manufactured by Toho Chemical Co., Ltd.), and 2 parts by weight of Dikssol W-92 (trade name of polyoxyethylene octylphenyl ether manufactured by Dai-ichi Kogyo Seiyaku Co., Ltd.).

Test Example 2

Corn (Zea mays) (varieties: Royal Dent 105T and Golden cross vantum) was sown in 6 test plots, each 2 $m^2$, and weeds were allowed to naturally grow therein. The growth stages of the plants 36 days after the sowing of the corn were 4 to 6-leaf stages (a 5.2-leaf stage on the average) for Royal Dent 105T, 3 to 5.6-leaf stages (a 4.2-leaf stage on the average) for Golden cross vantum, 1 to 7-leaf stages (a 4.5-leaf stage on the average) for green foxtail (Setaria viridis), 1 to 6-leaf stages (a 4-leaf stage on the average) for large crabgrass (Digitaria adscendens), 2 to 6.5-leaf stages (a 4-leaf stage on the average) for smartweed (Polygonum hydropiper), cotyledon to 4-leaf stages (a 2-leaf stage on the average) for pigweed (Amaranthus retroflexus), and cotyledon to 4.2-leaf stages (a 3-leaf stage on the average) for cocklebur (Xanthium strumarium). Thirty six days after sowing, 8 liters/a each of aqueous diluted suspensions prepared by suspending in water the suspension concentrate prepared in the aforementioned Formulation Example 3 (corn oil was used as vegetable oil) so as to provide respective amounts of the active ingredient of 1.25 g/a

and 0.75 g/a were foliarly applied to the plants with a manual knap-sack type sprayer. The foregoing test was repeated.

The progress of growth of the plants was visually observed and examined 45 days after the application to evaluate the degree of growth control according to the same ratings as in the aforementioned Test Example 1.

The average ratings for the degree of growth control evaluated for the two test runs were found to be 1 for both of the two varieties of corn as crops and 10 for all of green foxtail, large crabgrass, smartweed, pigweed, and cocklebur.

Test Example 3

1/3,000 are (a) pots were filled with upland soil. Corn (Zea mays) (variety: Royal Dent 105 T) and large crabgrass (Digitaria adscendens) were sown in the separate pots respectively, and grown under upland condition in a greenhouse. When the plants reached respective given growth stages (a 4.8-leaf stage for corn and a 3.5-leaf stage for large crabgrass), 5 liters/a each of aqueous diluted suspensions, prepared by suspending in water the suspension concentrate prepared in the aforementioned Formulation Example 3, were each separately foliarly applied to the plants with a small spray gun.

The progress of growth of the plants was visually observed and examined 28 days after the application to evaluate the degree of growth control according to the same ratings as in the aforementioned Test Example 1. The results are shown in Table 2.

For comparison, a test was made according to substantially the same procedure as in the above-mentioned Test Example 3 using the same wettable powder applied for the comparative plots in the aforementioned Test Example 1.

Table 2

| Vegetable Oil Used | Degree of Growth Control | | | | | |
|---|---|---|---|---|---|---|
| | corn 4.8L | | | large crabgrass 3.5L | | |
| | Amount of Active Ingredient (g/a) | | | | | |
| | 1 | 0.5 | 0.25 | 1 | 0.5 | 0.25 |
| Corn Oil<br>Plots Using Present Invention | 1 | 1 | 1 | 9-10 | 9 | 6 |
| Soybean Oil<br>Plots Using Present Invention | 1 | 1 | 1 | 10 | 9-10 | 6 |
| Cotton Seed Oil<br>Plots Using Present Invention | 1 | 1 | 1 | 9-10 | 9 | 7 |
| Rape Seed Oil<br>Plots Using Present Invention | 1 | 1 | 1 | 9-10 | 9 | 7-8 |
| Linseed Oil<br>Plots Using Present Invention | 1 | 1 | 1 | 10 | 9 | 6 |
| None<br>Plots Using Comparative Suspension | 1 | 1 | 1 | 4 | 3 | 2 |

**Claims**

**Claims for the following Contracting States : DE, FR, GB, IT**

**1.** A herbicidal suspension concentrate characterized by comprising from 0.5 to 20 parts by weight of at least one compound selected from among sulfonamide compounds represented by the general formula (I):

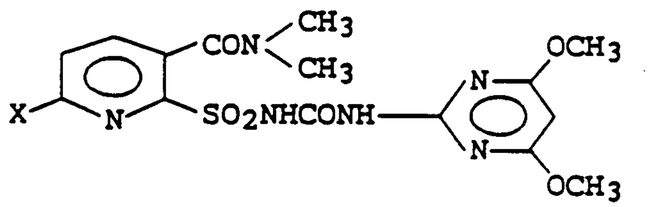

(I)

(wherein X is a hydrogen atom, a chlorine atom, a bromine atom, a methyl group, or a difluoromethyl group) and their salts as an active ingredient, from 55 to 94.5 parts by weight of a vegetable oil, and from 5 to 25 parts by weight of a surfactant.

**2.** A herbicidal suspension concentrate as claimed in claim 1, characterized in that the vegetable oil is at least one selected from the group consisting of olive oil, kapok oil, castor oil, palm oil, camellia oil, coconut oil, sesame oil, corn oil, rice bran oil, peanut oil, cotton seed oil, soybean oil, rape seed oil, linseed oil and tung oil.

**3.** A herbicidal suspension concentrate as claimed in claim 1, characterized in that the surfactant is at least one selected from the group consisting of surfactants of vegetable oil derivatives, nonionic surfactants, and anionic surfactants.

**4.** A herbicidal suspension concentrate as claimed in claim 1, characterized in that the weight ratio of the sulfonamide compound represented by the general formula (I) or its salt, the vegetable oil and the surfactant is in the range of from 2 to 6:from 77 to 90:from 8 to 17, respectively.

**5.** A herbicidal suspension concentrate as claimed in claim 1, characterized in that the suspension concentrate comprises from 0.5 to 20 parts by weight of N-[(4,6-dimethoxypyrimidin-2-yl)-aminocarbonyl]3-dimethylaminocarbonyl-2-pyridinesulfonamide or its salt, from 55 to 94.5 parts by weight of corn oil, and from 5 to 25 parts by weight of a mixture of surfactant of vegetable oil derivative, nonionic surfactant, and anionic surfactant.

**6.** A method for preparation of a herbicidal suspension concentrate characterized by comprising mixing from 0.5 to 20 parts by weight of at least one compound selected from among sulfonamide compounds represented by the general formula (I):

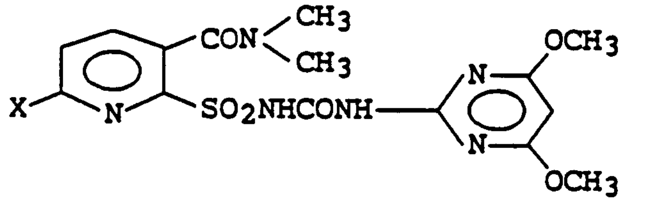

(I)

(wherein x is a hydrogen atom, a chlorine atom, a bromine atom, a methyl group, or a difluoromethyl group) and their salts as an active ingredient, from 55 to 94.5 parts by weight of a vegetable oil, and from 5 to 25 parts by weight of a surfactant.

**7.** A method as claimed in claim 6, wherein the vegetable oil is at least one selected from the group consisting of olive oil, kapok oil, castor oil, palm oil, camellia oil, coconut oil, sesame oil, corn oil, rice bran oil, peanut oil, cotton seed oil, soybean oil, rape seed oil, linseed oil and tung oil.

**8.** A method as claimed in claim 6 or 7, wherein the surfactant is at least one selected from the group

consisting of surfactants of vegetable oil derivatives, nonionic surfactants, and anionic surfactants.

**9.** A method as claimed in claim 6, 7 or 8, wherein the mixing is effected so that the weight ratio of the sulfonamide compound represented by the general formula (I) or its salt, the vegetable oil and the surfactant is in the range of from 2 to 6: from 77 to 90: from 8 to 17, respectively.

**10.** A method as claimed in claim 6, wherein the mixing is effected so that the suspension concentrate comprises from 0.5 to 20 parts by weight of N-[(4,6-dimethoxypyrimidin-2-yl)aminocarbonyl]-3-dimethylaminocarbonyl-2-pyridinesulfonamide or its salt, from 55 to 94.5 parts by weight of corn oil, and from 5 to 25 parts by weight of a mixture of surfactant of vegetable oil derivative, nonionic surfactant, and anionic surfactant.

**Claims for the following Contracting States : ES, GR**

**1.** A method for preparation of a herbicidal suspension concentrate characterized by comprising mixing from 0.5 to 20 parts by weight of at least one compound selected from among sulfonamide compounds represented by the general formula (I):

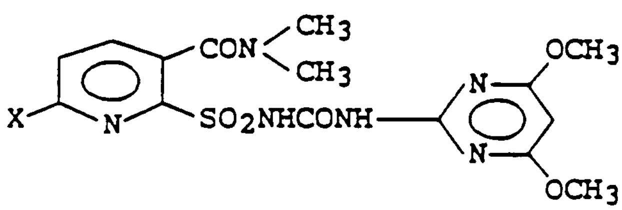

(I)

(wherein x is a hydrogen atom, a chlorine atom, a bromine atom, a methyl group, or a difluoromethyl group) and their salts as an active ingredient, from 55 to 94.5 parts by weight of a vegetable oil, and from 5 to 25 parts by weight of a surfactant.

**2.** A method as claimed in claim 1, wherein the vegetable oil is at least one selected from the group consisting of olive oil, kapok oil, castor oil, palm oil, camellia oil, coconut oil, sesame oil, corn oil, rice bran oil, peanut oil, cotton seed oil, soybean oil, rape seed oil, linseed oil and tung oil.

**3.** A method as claimed in claim 1 or 2, wherein the surfactant is at least one selected from the group consisting of surfactants of vegetable oil derivatives, nonionic surfactants, and anionic surfactants.

**4.** A method as claimed in claim 1, 2 or 3, wherein the mixing is effected so that the weight ratio of the sulfonamide compound represented by the general formula (I) or its salt, the vegetable oil and the surfactant is in the range of from 2 to 6: from 77 to 90: from 8 to 17, respectively.

**5.** A method as claimed in claim 1, wherein the mixing is effected so that the suspension concentrate comprises from 0.5 to 20 parts by weight of N-[(4,6-dimethoxypyrimidin-2-yl)aminocarbonyl]-3-dimethylaminocarbonyl-2-pyridinesulfonamide or its salt, from 55 to 94.5 parts by weight of corn oil, and from 5 to 25 parts by weight of a mixture of surfactant of vegetable oil derivative, nonionic surfactant, and anionic surfactant.

**Revendications**

**Revendications pour les Etats contractants suivants : DE, FR, GB, IT**

**1.** Un concentré de suspension herbicide caractérisé en ce qu'il comprend de 0,5 à 20 parties en poids d'au moins un composé choisi parmi les composés sulfonamides représentés par la formule générale (I) :

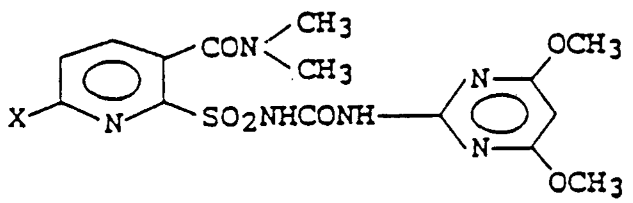

(I)

(dans laquelle X est un atome d'hydrogène, un atome de chlore, un atome de brome, un groupe méthyle, ou un groupe difluorométhyle) et leurs sels comme ingrédient actif, de 55 à 94,5 parties en poids d'une huile végétale, et de 5 à 25 parties en poids d'un surfactant.

2. Un concentré de suspension herbicide selon la revendication 1, caractérisé en ce que l'huile végétale est au moins une huile choisie dans le groupe comprenant l'huile d'olive, l'huile de kapok, l'huile de ricin, l'huile de palme, l'huile de camélia, l'huile de copra, l'huile de sésame, l'huile de mais, l'huile de son de riz, l'huile d'arachide, l'huile de graines de coton, l'huile de graines de soja, l'huile de colza, l'huile de lin et l'huile de tung.

3. Un concentré de suspension herbicide selon la revendication 1, caractérisé en ce que le surfactant est l'un au moins des surfactants choisis dans le groupe comprenant les surfactants dérivés d'huiles végétales, les surfactants non ioniques, et les surfactants anioniques.

4. Un concentré de suspension herbicide selon la revendication 1, caractérisé en ce que le rapport pondéral du composé sulfonamide représenté par la formule générale (I) ou de son sel, à l'huile végétale et au surfactant est dans l'intervalle de 2 à 6 : de 77 à 90 : de 8 à 17 respectivement.

5. Un concentré de suspension herbicide selon la revendication 1, caractérisé en ce que le concentré de suspension comprend de 0,5 à 20 parties en poids de N-[(4,6-diméthoxypyrimidine-2-yl)-aminocarbonyl]-3--diméthylaminocarbonyl--2-pyridinesulfonamide ou son sel, de 55 à 94,5 parties en poids d'huile de mais, et de 5 à 25 parties en poids d'un mélange de surfactants dérivés d'huiles végétales, de surfactant non ionique et de surfactant anionique.

6. Une méthode de préparation d'un concentré de suspension herbicide caractérisée en ce qu elle comprend le mélangeage de 0,5 à 20 parties en poids d'au moins un composé choisi parmi les composés sulfonamides représentés par la formule générale (I) :

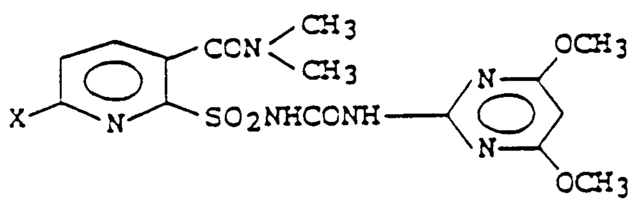

(I)

(dans laquelle X est un atome d'hydrogène, un atome de chlore, un atome de brome, un groupe méthyle ou un groupe difluorométhyle) et leurs sels comme ingrédient actif, de 55 à 94,5 parties en poids d'une huile végétale et de 5 à 25 parties en poids d'un surfactant.

7. Une méthode selon la revendication 6, selon laquelle l'huile végétale est l'une au moins des huiles choisies dans le groupe contenant l'huile d'olive, l'huile de kapok, l'huile de ricin, l'huile de palme, l'huile de camélia, l'huile de copra, l'huile de sésame, l'huile de mais, l'huile de son de riz, l'huile d'arachide, l'huile de graines de coton, l'huile de graines de soja, l'huile de colza, l'huile de lin et l'huile de tung.

8. Une méthode selon la revendication 6 ou 7, selon laquelle le surfactant est l'un au moins des surfactants choisis dans le groupe comprenant les surfactants dérivés d'huiles végétales, les surfactants non ioniques et les surfactants anioniques.

**9.** Une méthode selon la revendication 6, 7 ou 8, selon laquelle le mélangeage est effectué de façon que le rapport pondéral du composé sulfonamide représenté par la formule générale (I) ou son sel, à l'huile végétale et au surfactant est dans l'intervalle de 2 à 6 : de 77 à 90 : de 8 à 17 respectivement.

**10.** Une méthode selon la revendication 6, selon laquelle le mélangeage est effectué de façon que le concentré de suspension comprenne de 0,5 à 20 parties en poids de N-[(4,6-diméthoxy-Pyrimidine--2-yl)aminocarbonyl]-3-diméthylaminocarbonyl-2-pyridine-sulfonamide ou son sel, de 55 à 94,5 parties en poids d'huile de mais, et de 5 à 25 parties en poids d'un mélange de surfactant dérivé d'huiles végétales, de surfactant non ionique et de surfactant anionique.

**Revendications pour les Etats contractants suivants : ES, GR**

**1.** Une méthode de préparation d'un concentré de suspension herbicide caractérisée en ce qu'elle comprend le mélangeage de 0,5 à 20 parties en poids d'au moins un composé choisi parmi les composés sulfonamides représentés par la formule générale (I) :

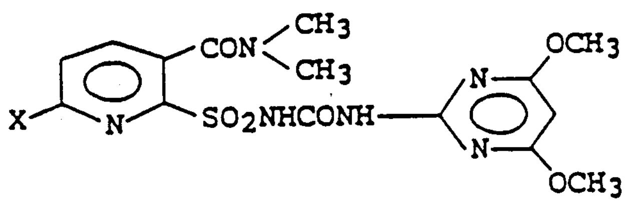

(I)

(dans laquelle X est un atome d'hydrogène, un atome de chlore, un atome de brome, un groupe méthyle ou un groupe difluorométhyle) et leurs sels comme ingrédient actif, de 55 à 94,5 parties en poids d'une huile végétale et de 5 à 25 parties en poids d'un surfactant.

**2.** Une méthode selon la revendication 1, selon laquelle l'huile végétale est l'une au moins des huiles choisies dans le groupe comprenant l'huile d'olive, l'huile de kapok, l'huile de ricin, l'huile de palme, l'huile de camélia, l'huile de copra, l'huile de sésame, l'huile de mais, l'huile de son de riz, l'huile d'arachide, l'huile de graines de coton, l'huile de graines de soja, l'huile de colza, l'huile de lin et l'huile de tung.

**3.** Une méthode selon la revendication 1 ou 1, selon laquelle le surfactant est l'un au moins des surfactants choisis dans le groupe comprenant les surfactants dérivés d'huiles végétales, les surfactants non ioniques et les surfactants anioniques.

**4.** Une méthode selon la revendication 1, 2 ou 3, selon laquelle le mélangeage est effectué de façon que le rapport pondéral du composé sulfonamide représenté par la formule générale (I) ou son sel, à l'huile végétale et au surfactant est dans l'intervalle de 2 à 6 : de 77 à 90 : de 8 à 17 respectivement.

**5.** Une méthode selon la revendication 1, selon laquelle le mélangeage est effectué de façon que le concentré de suspension comprenne de 0,5 à 20 parties en poids de N-[(4,6-diméthoxy-Pyrimidine--2-yl)aminocarbonyl]-3-diméthylaminocarbonyl-2-pyridine-sulfonamide ou son sel, de 55 à 94,5 parties en poids d'huile de mais, et de 5 à 25 parties en poids d'un mélange de surfactant dérivé d'huiles végétales, de surfactant non ionique et de surfactant anionique.

**Patentansprüche**

**Patentansprüche für folgende Vertragsstaaten : DE, FR, GB, IT**

**1.** Herbizides Suspensionskonzentrat,
dadurch **gekennzeichnet,** daß
es 0,5 bis 20 Gew.Teile zumindest einer Verbindung, ausgewählt aus Sulfonamidverbindungen, die durch die allgemeine Formel (I) dargestellt sind:

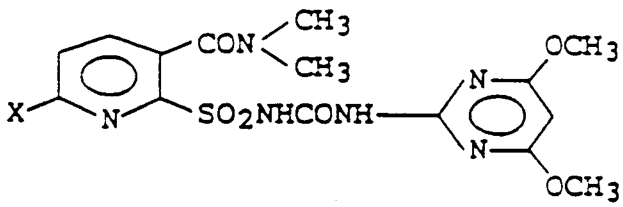

(I)

worin X ein Wasserstoffatom, ein Chloratom, ein Bromatom, eine Methylgruppe oder eine Difluoromethylgruppe darstellt und deren Salze als aktiven Bestandteil, 55 bis 94,5 Gew.Teile eines Pflanzenöls und 5 bis 25 Gew.Teile eines oberflächenaktiven Mittels enthält.

2. Herbizides Suspensionskonzentrat nach Anspruch 1,
dadurch **gekennzeichnet,** daß
das Pflanzenöl zumindest ein Öl ist, ausgewählt aus der Gruppe, die aus Olivenöl, Kapoköl, Castoröl, Palmöl, Camelliaöl, Kokosnußöl, Sesamöl, Maisöl, Reiskleieöl, Erdnußöl, Baumwollsamenöl, Sojabohnenöl, Rapssamenöl, Leinsamenöl und Tangöl besteht.

3. Herbizides Suspensionskonzentrat nach Anspruch 1,
dadurch **gekennzeichnet,** daß
das oberflächenaktive Mittel zumindest ein Mittel ist ausgewählt aus der Gruppe, bestehend aus oberflächenaktiven Mitteln von Pflanzenölderivaten, nicht ionischen oberflächenaktiven Mitteln und anionischen oberflächenaktiven Mitteln.

4. Herbizides Suspensionskonzentrat nach Anspruch 1,
dadurch **gekennzeichnet,** daß
das Gewichtsverhältnis der Sulfonamidverbindung, die durch die allgemeine Formel (I) dargestellt ist, oder deren Salz, des Pflanzenöls und des oberflächenaktiven Mittels in dem Bereich von 2 bis 6: 77 bis 90 : 8 bis 17 liegt.

5. Herbizides Suspensionskonzentrat nach Anspruch 1,
dadurch **gekennzeichnet,** daß
das Suspensionskonzentrat 0,5 bis 20 Gew.Teile N((4,6-Dimethoxypyrimidin-2-yl)aminocarbonyl)-3-dimethylaminocarbonyl-2-pyridinsulfonamid oder dessen Salz, 55 bis 94,5 Gew.Teile Maisöl und 5 bis 25 Gew.Teile einer Mischung aus einem oberflächenaktiven Mittel eines Pflanzenölderivates, nichtionischen oberflächenaktiven Mittels und anionischen oberflächenaktiven Mittels enthält.

6. Verfahren zur Herstellung eines herbiziden Suspensionskonzentrates,
**gekennzeichnet** durch
Mischen von 0,5 bis 20 Gew.Teilen zumindest einer Verbindung, die aus Sulfonamidverbindungen ausgewählt ist, die durch die allgemeine Formel (I) dargestellt sind:

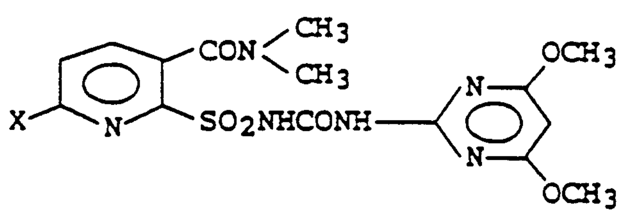

(I)

worin X ein Wasserstoffatom, ein Chloratom, ein Bromatom, eine Methylgruppe oder eine Difluoromethylgruppe ist und deren Salzen als aktiven Bestandteil, 55 bis 94,5 Gew.Teilen eines Pflanzenöls und 5 bis 25 Gew.Teilen eines oberflächenaktiven Mittels.

7. Verfahren nach Anspruch 6,
dadurch **gekennzeichnet,** daß
das Pflanzenöl zumindest ein Öl ist, ausgewählt aus der Gruppe, die aus Olivenöl, Kapoköl, Castoröl, Palmöl, Camelliaöl, Kokosnußöl, Sesamöl, Maisöl, Reiskleieöl, Erdnußöl, Baumwollsamenöl, Sojabohnenöl, Rapssamenöl, Leinsamenöl und Tungöl besteht.

**8.** Verfahren nach Anspruch 6 oder 7, dadurch **gekennzeichnet,** daß das oberflächenaktive Mittel zumindest ein Mittel ist, das aus der Gruppe ausgewählt wird, die aus oberflächenaktiven Mitteln von Pflanzenölderivaten, nicht ionischen oberflächenaktiven Mitteln und anionischen oberflächenaktiven Mitteln besteht.

**9.** Verfahren nach Anspruch 6, 7 oder 8, dadurch **gekennzeichnet,** daß das Mischen so durchgeführt wird, daß das Gewichtsverhältnis der Sulfonamidverbindung, die durch die allgemeine Formel (I) dargestellt ist, oder deren Salz, des Pflanzenöls und des oberflächenaktiven Mittels in dem Bereich von 2 bis 6 : 77 bis 90 : 8 bis 17 liegt.

**10.** Verfahren nach Anspruch 6, dadurch **gekennzeichnet,** daß das Mischen so durchgeführt wird, daß das Suspensionskonzentrat 0,5 bis 20 Gew.Teile N-((4,6-Dimethoxypyrimidin-2-yl)aminocarbonyl)-3-dimethylaminocarbonyl-2-pyridinsulfonamid oder dessen Salz, 55 bis 94,5 Gew.Teile Maisöl und 5 bis 25 Gew.Teile einer Mischung eines oberflächenaktiven Mittels von einem Pflanzenölderivat, nichtionischen oberflächenaktiven Mitteln und anionischen oberflächenaktiven Mitteln enthält.

**Patentansprüche für folgende Vertragsstaaten : ES, GR**

**1.** Verfahren zur Herstellung eines herbiziden Suspensionskonzentrates, **gekennzeichnet** durch Mischen von 0,5 bis 20 Gew.Teilen zumindest einer Verbindung, die aus Sulfonamidverbindungen ausgewählt ist, die durch die allgemeine Formel (I) dargestellt sind:

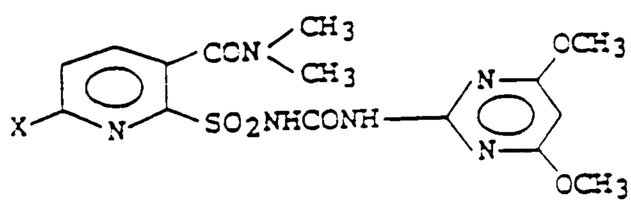

(I)

worin X ein Wasserstoffatom, ein Chloratom, ein Bromatom, eine Methylgruppe oder eine Difluoromethylgruppe ist und deren Salzen als aktiven Bestandteil, 55 bis 94,5 Gew.Teilen eines Pflanzenöls und 5 bis 25 Gew.Teilen eines oberflächenaktiven Mittels.

**2.** Verfahren nach Anspruch 1, dadurch **gekennzeichnet,** daß das Pflanzenöl zumindest ein Öl ist, ausgewählt aus der Gruppe, die aus Olivenöl, Kapoköl, Castoröl, Palmöl, Camelliaöl, Kokosnußöl, Sesamöl, Maisöl, Reiskleieöl, Erdnußöl, Baumwollsamenöl, Sojabohnenöl, Rapssamenöl, Leinsamenöl und Tungöl besteht.

**3.** Verfahren nach Anspruch 1 oder 2, dadurch **gekennzeichnet,** daß das oberflächenaktive Mittel zumindest ein Mittel ist, das aus der Gruppe ausgewählt wird, die aus oberflächenaktiven Mitteln von Pflanzenölderivaten, nicht-ionischen oberflächenaktiven Mitteln und anionischen oberflächenaktiven Mitteln besteht.

**4.** Verfahren nach Anspruch 1, 2 oder 3, dadurch **gekennzeichnet,** daß das Mischen so durchgeführt wird, daß das Gewichtsverhältnis der Sulfonamidverbindung, die durch die allgemeine Formel (I) dargestellt ist, oder deren Salz, des Pflanzenöls und des oberflächenaktiven Mittels in dem Bereich von 2 bis 6 : 77 bis 90 : 8 bis 17 liegt.

**5.** Verfahren nach Anspruch 1,
dadurch **gekennzeichnet,** daß
das Mischen so durchgeführt wird, daß das Suspensionskonzentrat 0,5 bis 20 Gew.Teile N-((4,6-Dimethoxypyrimidin-2-yl)aminocarbonyl)-3-dimethylaminocarbonyl-2-pyridinsulfonamid oder dessen Salz, 55 bis 94,5 Gew.Teile Maisöl und 5 bis 25 Gew.Teile einer Mischung eines oberflächenaktiven Mittels von einem Pflanzenölderivat, nichtionischen oberflächenaktiven Mitteln und anionischen oberflächenaktiven Mitteln enthält.